# EUROPEAN PATENT SPECIFICATION

(11) **EP 1 799 194 B1**
(45) Date of publication and mention of the grant of the patent: **16.11.2011**
(21) Application number: 05813897.5
(22) Date of filing: 16.09.2005
(51) Int. Cl.: A61K 9/14

(54) **A NEW CLASS OF SURFACTANT-LIKE MATERIALS COMPRISING VITAMIN E TPGS AND A WATER SOLUBLE POLYMER**
NEUE KLASSE VON SURFUCTANTÄHNLICHEN MATERIALIEN MIT VITAMIN-E-TPGS UND WASSERLÖSLICHEM POLYMER
NOUVELLE CLASSE DE MATERIAUX DE TYPE TENSIOACTIF COMPRENANT DU VITAMINE E TPGS ET DU POLYMERE HYDROSOLUBLE

(30) Priority: 24.09.2004 US 612716 P
(43) Date of publication of application: 27.06.2007
(73) Proprietor: Boehringer Ingelheim Pharmaceuticals Inc., Ridgefield, Connecticut 06877-0368 (US)
(72) Inventor: LI, Jinjiang, Ridgefield, CT 06877-0368 (US); CHEN, Shirlynn, Ridgefield, CT 06877-0368 (US); GEREG, George W., Jr., Ridgefield, CT 06877-0368 (US); CORSON, Mariotte, Ridgefield, CT 06877-0368 (US)
(74) Representative: Hammann, Heinz
(86) International application number: PCT/US2005/033324
(87) International publication number: WO 2006/036614

(56) References cited:
- US-A- 5 558 876
- US-B1- 6 416 793
- SETHIA S ET AL: "Solid dispersion of carbamazepine in PVP K30 by conventional solvent evaporation and supercritical methods." INTERNATIONAL JOURNAL OF PHARMACEUTICS (KIDLINGTON), vol. 272, no. 1-2, 19 March 2004 (2004-03-19), pages 1-10, XP002395638 ISSN: 0378-5173
- MU L ET AL: "A novel controlled release formulation for the anticancer drug paclitaxel (Taxol<(>R)): PLGA nanoparticles containing vitamin E TPGS" JOURNAL OF CONTROLLED RELEASE, ELSEVIER, AMSTERDAM, NL, vol. 86, no. 1, 9 January 2003 (2003-01-09), pages 33-48, XP004398997 ISSN: 0168-3659
- SHIN SANG-CHUL ET AL: "Physicochemical characterization of solid dispersion of furosemide with TPGS." INTERNATIONAL JOURNAL OF PHARMACEUTICS (KIDLINGTON), vol. 251, no. 1-2, 30 January 2003 (2003-01-30), pages 79-84, XP001125854 ISSN: 0378-5173

## Description

### TECHNICAL FIELD

This invention generally refers to a novel class of surfactant-like material that promotes the solubility of poorly soluble compounds.

### BACKGROUND OF THE INVENTION

Despite extensive research efforts, solubility and dissolution rate remain key problems in drug discovery and product development for oral dosage forms (Mehta, Bulletin Tech., Gattefose, No. 91, 65-71 (1998)). Compounds that have limited solubility, in water, typically below 0.1 mg/ml, present unusual challenges in drug discovery (Wyatt, Bulletin Tech Gattefose, No. 92, 31-39 (1999)). This is especially true in circumstances where solubility and dissolution limit drug absorption.

Strategies for improving apparent solubility and dissolution rates include:
(a) forming soluble salts for ionizable drugs (Nelson, J. Pharm. Sci., 47, 297 (1958));
(b) reducing crystal size (Nelson, J. Pharm. Sci., 53, 1224 (1964));
(c) forming soluble pro-drugs;
(d) using amorphous forms (Niazi, J. Pharm. Sci., 65, 302 (1976));
(e) using co-solvents and superdisintegrants (Serajuddin, J. Pharm. Sci., 77(4), 325-329 (1998));
(f) impregnating liquid drugs or drug solution in porous powders (Jaworski, Drug Development and Industrial Pharmacy, 16(5), 965-977 (1990)); and
(g) using surface active self-emulsifying systems (Dordunoo, Drug Development and Industrial Pharmacy, 17(12), 1685-1713 (1991)).

Although salt formation and particle size reduction are commonly used to increase dissolution rate and oral absorption, there are practical limitations for these techniques. Salt formation is not feasible for neutral compounds and weak electrolytes. Very fine powders of hydrophobic drugs, on the other hand, are difficult to disperse in water due to the poor wettability of the particle surfaces (Seajuddin, J. Pharm. Sci., 88(10), 1058-1066 (1999)). The loading of liquid drugs in porous powder (called "powder solutions" by Jarowski, identified supra) encountered flow property and compressibility problems in pharmaceutical manufacture, consequently, it is only suitable for low-dose drugs. Among the strategies, those that increase dissolution rate of poorly soluble drugs with lipids/surfactants are the most commonly used techniques for enhancing their absorption.

For lipid-based dissolution enhancement, poorly soluble drugs are first dissolved in liquid lipid-melts and formulated as soft or hard capsules (Dordunoo, Drug Development and Industrial Pharmacy, 17(12), 1685-1713 (1991) and Seajuddin, J. Pharm. Sci., 88(10), 1058-1066 (1999)). This lipid-based hard gel capsule technology overcomes the scale-up difficulties in solid dispersion as well as avoids the drop in dissolution rate due to the loss of solubilizer and formation of a drug-rich surface layer. However, when the shell of such a capsule disintegrates after oral ingestion, the drug-lipid matrix is exposed to the gastrointestinal fluid as a solid plug, and drug dissolution may be limited by surface erosion of this solid plug. Self-emulsifying powders were also prepared by freeze-drying drug-lipid emulsions or microemulsions (Pather, U.S. Patent No. 6,280,770, 2001). Similarly, insoluble drugs also form microemulsion with lipids. These microemulsions are then freeze-dried to provide the solid dosage form.(Lundberg, J. Pharm. Pharmacol., 49, 16-21 (1997)). However, these processes encounter either scale-up complexity or the need of organic solvents.

Vitamin E TPGS (D-alpha-tocopherol PEG 1000 succinate) 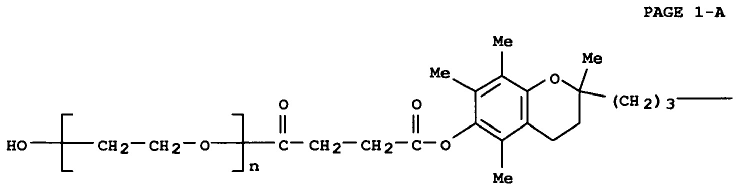 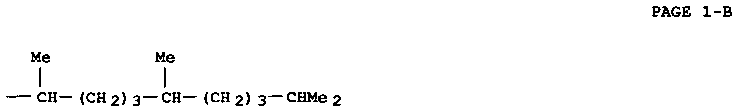 (hereinafter "VeTPGS") is a nonionic surfactant which is useful as an emulsifier, in liquid formulations such as hydrophobic compounds, through micellization. VeTPGS has been used in a drug composition to form a true molecular solution to provide a slowly dissolving TPGS/drug matrix that absorbs gastrointestinal fluid into the matrix at the dosage form/fluid interface, where a gel-like liquid crystal is formed.(US 5,891,845).

Various mechanisms have been employed to improve the solubility of poorly water soluble compounds using VeTPGS. Mesoporous compositions for use in drug delivery are discussed in US 2002/0164380 A1; stability of polyethylene oxide in matrix tablets prepared by hot-melt extrusion is disclosed in Crowley (Biomaterials 23 (2002) 4241-4248); VeTPGS used as emulsifier in solvent evaporation/extraction technique for fabrication of polymeric nanosphere for controlled release of paclitaxel (Taxol®) in Mu, et al (Journal of Controlled Release, 80 (2002) 129-144); VeTPGS is used in the solvent extraction/evaporation technique for fabrication of polymeric nanosheres of an antineoplastic drug paclitaxel (Taxol®) for cancer therapy in Mu, et al (Bioengineering Conference, Vol. 50 (2001), 187-188); oxidized cellulose and vitamin E blend for topical hemostatic applications in Wu, et al (WO 97/38737); compositions and methods utilizing liquid crystal structures in Myers, et al (US Patent 5,891,845); the influence of VeTPGS on the properties of hydrophilic films produced by hot-melt extrusion in Repka, et al (International Journal of Pharmaceutics, 202 (2002), 63-70); and VeTPGS has been applied in polymeric compositions suitable for injection molding of a single or multi-component pharmaceutical dosage forms in WO 02/060382. However, in all these mechanisms, overcoming the waxy nature of VeTPGS, which causes processing problems such as flow, mixing, content uniformity, and its low Tm have not been addressed or resolved.

The instant invention overcomes the waxy nature of VeTPGS and provides for its emulsifying activity in solid formulations resulting in improving, enhacing and/or increasing the solubility and bioavailability of poorly water soluble compounds.

### SUMMARY OF THE INVENTION

The above-captioned technical problems with the use of VeTPGS alone in increasing the solubility of poorly soluble compounds is solved by the embodiments characterized in the claims and the description. The before-mentioned disadvantages in the art are overcome by the present invention as discussed in the description section.

The present invention provides a surfactant-like material wherein VeTPGS is converted into a truly solid material with surfactant property which can be utilized in solid dosage formulations. The invention also provides for methods of preparing this surfactant-like material.

### BRIEF DESCRIPTION OF THE FIGURES

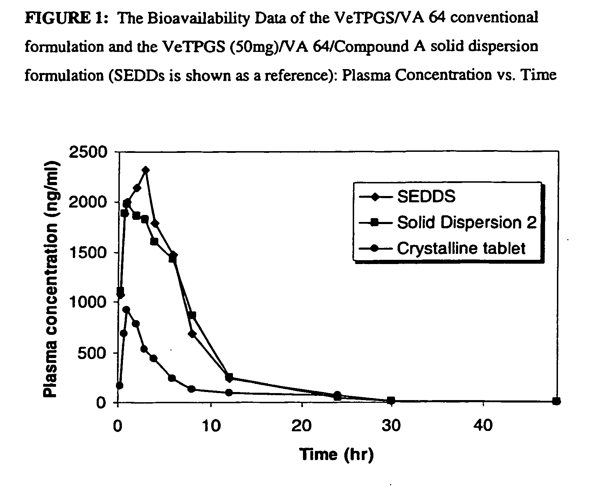

FIG. 1. Bioavailability profile of SEEDs, and VeTPGS/VA 64/ Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.04,6]nonadec-7-ene-4-carboxylic acid amorphous solid dispersion and Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.04,6]nonadec-7-ene-4-carboxylic acid (crystalline) with VeTPGS/Va64 dispersion.
FIG. 2. PXRD pattern of VeTPGS/VA 64/solid dispersion,(Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.04,6]nonadec-7-ene-4-carboxylic acid VA 64 and VeTPGS.
FIG. 3. Bioavailability data of VeTPGS/VA 64 conventional formulations versus other formulations.

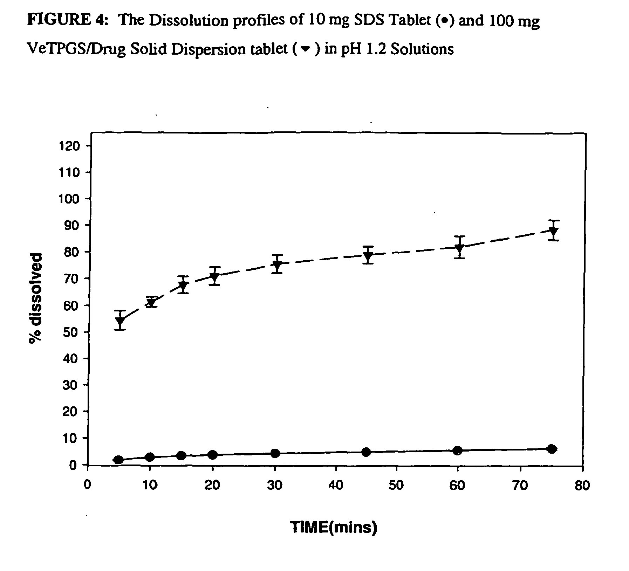

FIG. 4. SDS versus VeTPGS/drug solid dispersion tablet.
FIG. 5. Dissolution profiles of VeTPGS/VA 64, VeTPGS/PVP K17, VeTPGS/Drug substance and 10 mg of SDS.

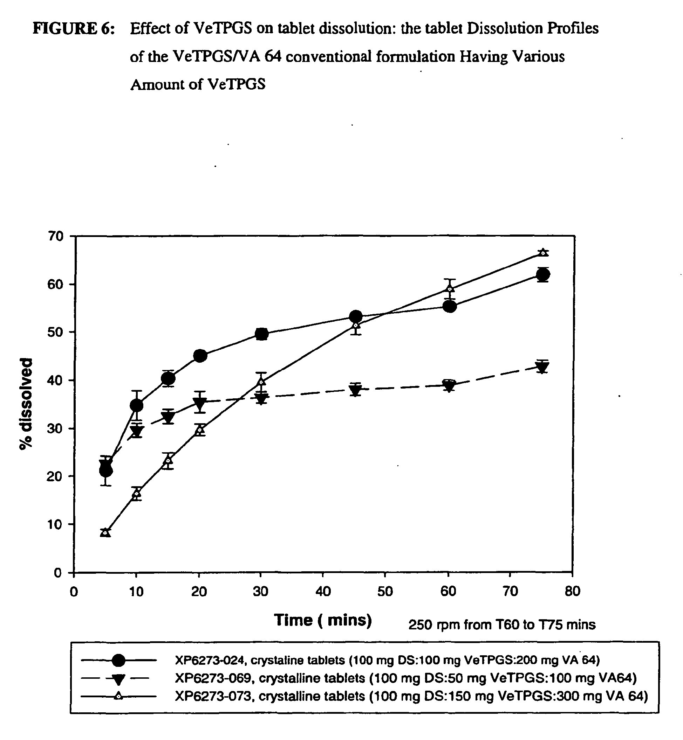

FIG. 6. Effect of VeTPGS amounts on tablet dissolution.

### DETAILED DESCRIPTION OF THE INVENTION

Terms not specifically defined herein should be given the meanings that would be given to them by one of skill in the art in light of the disclosure and the context. As used in the specification and appended claims, however, unless specified to the contrary, the following terms have the meaning indicated and the following conventions are adhered to.

Before the embodiments of the present invention, it must be noted that as used herein and in the appended claims, the singular "a", "an", and "the" include plural reference unless the context clearly dictates otherwise. Thus, for example, reference to "a surfactant-like material" includes a plurality of such surfactant-like materials, reference to "a water soluble polymer" is a reference to one or more such water soluble polymers and equivalents thereof known to those skilled in the art, and so forth.

In the present invention it was found that surfactant-like material powder of the instant invention has demonstrated improved solubility, dissolution rate and absorption of drugs which are poorly soluble.

The present invention relates to a surfactant-like material comprising Vitamin E TPGS and at least one water soluble polymer and optionally additional carrier or excipients.

Another object of the invention is a method of preparing the surfactant-like material comprising Vitamin E TPGS and at least one water soluble polymer and optionally additional carrier or excipients, comprising the steps of:
(a) dissolving Vitamin E TPGS and a water soluble polymer in a solvent system; and
(b) employing drying technology till the resultant material is a free-flowing powder.

Another object of the invention is a method of preparing the surfactant-like material comprising Vitamin E TPGS and at least one water soluble polymer and optionally additional carrier or excipients, comprising the steps of:
(a) dissolving Vitamin E TPGS and a water soluble polymer in a solvent system; and
(b) employing drying technology till the resultant material is a free-flowing powder,
wherein the ratio of water soluble polymer to Vitamin E TPGS is in the range of about 2:1 to about 15:1, preferably in the range of about 2:1 to about 5:1.

Another object of the invention is a surfactant-like material prepared by the process comprising the steps of:
(a) dissolving Vitamin E TPGS and a water soluble polymer in a solvent system; and
(b) employing drying technology till the resultant material is a free-flowing powder.

Another object of the invention is surfactant-like material prepared by a process comprising the steps of:
(a) dissolving Vitamin E TPGS and a water soluble polymer in a solvent system; and
(b) employing drying technology till the resultant material is a free-flowing powder,
wherein the ratio of water soluble polymer to Vitamin E TPGS is in the range of about 2:1 1 to about 15:1, preferably in the range of about 2:1 to about 5:1.

Another object of the invention is a surfactant-like material comprising Vitamin E TPGS and at least one water soluble polymer and optionally additional carrier or excipients that does not contain a therapeutically active agent or a tautomer, , solvate or salt thereof

Another aspect of the invention is where the surfactant-like material is a homogeneous, amorphous or semi-crystalline powder comprising Vitamin E TPGS and at least one water soluble polymer and optionally additional carrier or excipients.

Another aspect of the invention is where the surfactant-like material is a homogenous powder comprising Vitamin E TPGS and at least one water soluble polymer and optionally additional carrier or excipients.

Another aspect of the invention is where the surfactant-like material is an amorphous powder comprising Vitamin E TPGS and at least one water soluble polymer and optionally additional carrier or excipients.

Another aspect of the invention is where the surfactant-like material is a semi-crystalline powder comprising Vitamin E TPGS and at least one water soluble polymer and optionally additional carrier or excipients.

Another object of the invention is a method of preparing the surfactant-like material which is a homogeneous, amorphous or semi-crystalline powder comprising Vitamin E TPGS and at least one water soluble polymer and optionally additional carrier or excipients, comprising the steps of:
(a) dissolving Vitamin E TPGS and a water soluble polymer in a solvent system; and
(b) employing drying technology till the resultant material is a free-flowing powder.

Another object of the invention is a method of preparing the surfactant-like material which is a homogeneous, amorphous or semi-crystalline powder comprising Vitamin E TPGS and at least one water soluble polymer and optionally additional carrier or excipients, comprising the steps of:
(a) dissolving Vitamin E TPGS and a water soluble polymer in a solvent system; and
(b) employing drying technology till the resultant material is a free-flowing powder,
wherein the ratio of water soluble polymer to Vitamin E TPGS is in the range of about 2:1 to about 15:1, preferably in the range of about 2:1 to about 5:1.

Another object of the invention is a surfactant-like material which is a homogeneous, amorphous or semi-crystalline powder prepared by the process comprising the steps of:
(a) dissolving Vitamin E TPGS and a water soluble polymer in a solvent system; and
(b) employing drying technology till the resultant material is a free-flowing powder.

Another object of the invention is surfactant-like material which is a homogeneous, amorphous or semi-crystalline powder prepared by a process comprising the steps of:
(a) dissolving Vitamin E TPGS and a water soluble polymer in a solvent system; and
(b) employing drying technology till the resultant material is a free-flowing powder,
wherein the ratio of water soluble polymer to Vitamin E TPGS is in the range of about 2:1 to about 15:1, preferably in the range of about 2:1 to about 5:1.

Another object of the invention is a surfactant-like material is a homogenous, amorphous or semi-crystalline powder comprising Vitamin E TPGS and at least one water soluble polymer and optionally additional carrier or excipients does not contain a therapeutically active agent or a tautomer, , solvate or salt thereof

Another object of the invention is a method of preparing the composition comprising surfactant-like material which is a homogenous, amorphous or semi-crystalline powder comprising Vitamin E TPGS and at least one water soluble polymer and a poorly soluble active agent or a tautomer, , solvate or salt therof and optionally a pharmaceutically acceptable carriers or excipients comprising the steps of:
a. Dissolving the VeTPGS and the water soluble polymer in a solvent system; and
b. Employing drying technology till the resultant material a free-flowing powder;
c. Mixing the dispersion with a poorly soluble active agentand optionally other excipients followed by a direct compression or by wet granulation and drying; and
d. Optionally followed by mixing granules with other tablet excipients for tableting

### DEFINITIONS

The term "homogenous" as used herein is defined as being uniform in kind, structure or composition.

The term "amorphous" as used herein is defined as being as lacking a long range order, non-crystalline or devoid of real or apparent crystalline form.

The term "water soluble polymer" as used herein may be selected from a group such as polyvinylpyrrolidones (PVP), copolyvidones, hydroxypropylmethylcellulose (HPMC); polyvinylalcohols, poly(methyl methacrylate), polysaccharides, polylactides, polyesters, and the like.

The preferred water soluble polymers include polyvinylpyrrolidones and copolyvidone VA 64, HPMC, and the like.

Examples of polyvinylpyrrolidones include but are not limited to Kollidon 12 PF, Kollidon 17 PF (PVP K17), Kollidon 25, Kollidon 30 (PVP K30), Kollidon 90 F, and the like.

Examples of copolyvidones include but are not limited to Kollidon VA 64 (VA 64) and the like.

Most preferred water soluble polymers include PVP K17, PVP K30 and VA 64.

The ratio of water soluble polymer to VeTPGS to be employed in the surfactant-like material is the range from about 2:1 to about 15:1, preferable in the range from about 2:1 to about 5:1.

The term "poorly soluble active agent" as used herein is defined as the solubility of compounds due to which solubilization becomes the rate limiting step for absorption of the compound. As a rule of thumb in pharmaceutical industry, such dissolution-related absorption problems cannot be rule out for a compound with an aqueous solubility less than 0.1/mL These poorly soluble compounds, new chemical entities, belong to Class II or below, that is, compounds that have an aqueous solubility of less that 0.1 mg/mL. Some examples of such poorly soluble active agents include but are not limited to Griseofulvin, Diazepam, Davazol, Cinnarixine, Halofantrine, Diclofenac, Etodolac, Indomethacin, Ketorolac, Sulindac, Tolmetin, Fenoprofen, Flurbiprofen, Ibuprofen, Ketoprofen, Naproxen, Oxaprozin, Mefanamic Acid, Acetyl-salicylic acid, Diflunisal, Salicyclic acid, Meloxicam, Prioxicam, Celecoxib, Rofecoxib, cyclosporine, triamterene, acyclovir, doxorubicin, labetalol, doxepin, methyldopa, (Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.0^{4,6}]nonadec-7-ene-4-carboxylic acid, pentoxifill and the like.

The preferred poorly soluble active agent being selected from Griseofulvin, Diazepam, Davazol, Cinnarixine, Halofantrine, Diclofenac, Etodolac, Indomethacin, Ketorolac, Sulindac, Tolmetin, Fenoprofen, Flurbiprofen, Ibuprofen, Ketoprofen, Naproxen, Oxaprozin, Mefanamic Acid, Acetyl-salicylic acid, Diflunisal, Salicyclic acid, Meloxicam, Prioxicam, Celecoxib, Rofecoxib, cyclosporine, triamterene, acyclovir, doxorubicin, labetalol, doxepin, methyldopa, pentoxifill and (Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.0^{4,6}]nona-dec-7-ene-4-carboxylic acid.
either an organic solvent or an aqueous media. Still more preferred poorly soluble active agentbeing (Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.0^{4,6}]nonadec-7-ene-4-carboxylic acid , whose chemical structure is as follows: 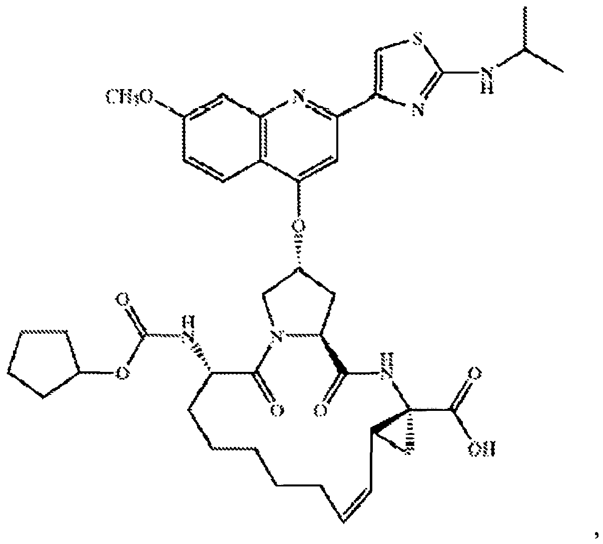 provided for in Tsantrizos et al., U.S. Patent No. 6,608,027 B1, which is hereby incorporated by reference.

The term "solvent system" as used herein is defined as either an organic solvent, a mixture of organic solvents or an aqueous media.

The term "drying technologies" as used herein is defined as technologies commonly employed to dry, such as spray-drying, freeze-drying, mechanical melt extrusion, etc. A reference on these technologies are disclosed in Advanced Drying Technologies, authored by Tadeusz Kudra, 2001, hereby incorporated by reference.

The term "excipients" as used herein is defined in the Handbook of Pharmaceutical Excipients, 2003 by Raymond Rowe, hereby incorporated by reference.

The terms "optional" or "optionally" mean that the subsequently described event or circumstances may or may not occur, and that the description includes instances where the event or circumstance occurs and instances in which it does not.

The term "salt" means an ionic form of the parent compound or the product of the reaction between the parent compound with a suitable acid or base to make the acid salt or base salt of the parent compound. Salts of the compounds of the present invention can be synthesized from the parent compounds which contain a basic or acidic moiety by conventional chemical methods. Generally, the salts are prepared by reacting the free base or acid parent compound with stoichiometric amounts or with an excess of the desired salt-forming inorganic or organic acid or base in a suitable solvent or various combinations of solvents.

The term "pharmaceutically acceptable salt" means a salt of a compound of the invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. As the compounds of the present invention are useful in both free base and salt form, in practice, the use of the salt form amounts to use of the base form. Lists of suitable salts are found in, e.g., S.M. Birge et al., J. Pharm. Sci., 1977, 66, pp. 1-19, which is hereby incorporated by reference in its entirety.

The term "pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid, sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, pyruvic acid, salicylic acid, stearic acid, succinic acid, sulfanilic acid, tartaric acid, *p-*toluenesulfonic acid, undecanoic acid, and the like.

The term "pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, *N*-ethylpiperidine, tetramethylammonium compounds, tetraethylammonium compounds, pyridine, *N*,*N-*dimethylaniline, *N*-methylpiperidine, *N*-methylmorpholine, dicyclohexylamine, dibenzylamine, *N*,*N*-dibenzylphenethylamine, 1-ephenamine, *N*,*N*'-dibenzylethylenediamine, polyamine resins, and the like. Particularly preferred organic nontoxic bases are isopropylamine, diethylamine, ethanolamine, trimethylamine, dicyclohexylamine, choline, and caffeine.

The term "solvate" means a physical association of a compound with one or more solvent molecules or a complex of variable stoichiometry formed by a solute and a solvent, for example, water, ethanol, or acetic acid. This physical association may involve varying degrees of ionic and other bondings, including hydrogen bonding. In certain instances, the solvate will be capable of isolation, for example, when one or more solvent molecules are incorporated in the crystal lattice of the crystalline solid. In general, the solvents selected do not interfere with the biological activity of the solute. Solvates encompasses both solution-phase and isolatable solvates. Representative solvates include hydrates, ethanolates, methanolates, and the like.

The term "hydrate" means a solvate wherein the solvent molecule(s) is/are H₂O.

The compounds of the present invention as discussed below include the free base or acid thereof, their salts, solvates, and s and may include oxidized sulfur atoms or quaternized nitrogen atoms in their structure, although not explicitly stated or shown, particularly the pharmaceutically acceptable forms thereof. Such forms, particularly the pharmaceutically acceptable forms, are intended to be embraced by the appended claims.

The term "patient" includes both human and non-human mammals.

The term "effective amount" means an amount of a compound according to the invention which, in the context of which it is administered or used, is sufficient to achieve the desired effect or result. Depending on the context, the term effective amount may include or be synonymous with a pharmaceutically effective amount or a diagnostically effective amount.

The terms "pharmaceutically effective amount" or "therapeutically effective amount" means an amount of a compound according to the invention which, when administered to a patient in need thereof, is sufficient to effect treatment for disease-states, conditions, or disorders for which the compounds have utility. Such an amount would be sufficient to elicit the biological or medical response of a tissue, system, or patient that is sought by a researcher or clinician. The amount of a compound of according to the invention which constitutes a therapeutically effective amount will vary depending on such factors as the compound and its biological activity, the composition used for administration, the time of administration, the route of administration, the rate of excretion of the compound, the duration of treatment, the type of disease-state or disorder being treated and its severity, drugs used in combination with or coincidentally with the compounds of the invention, and the age, body weight, general health, sex, and diet of the patient. Such a therapeutically effective amount can be determined routinely by one of ordinary skill in the art having regard to their own knowledge, the prior art, and this disclosure.

The term "diagnostically effective amount" means an amount of a compound according to the invention which, when used in a diagnostic method, apparatus, or assay, is sufficient to achieve the desired diagnostic effect or the desired biological activity necessary for the diagnostic method, apparatus, or assay. Such an amount would be sufficient to elicit the biological or medical response in a diagnostic method, apparatus, or assay, which may include a biological or medical response in a patient or in a in vitro or in vivo tissue or system, that is sought by a researcher or clinician. The amount of a compound according to the invention which constitutes a diagnostically effective amount will vary depending on such factors as the compound and its biological activity, the diagnostic method, apparatus, or assay used, the composition used for administration, the time of administration, the route of administration, the rate of excretion of the compound, the duration of administration, drugs and other compounds used in combination with or coincidentally with the compounds of the invention, and, if a patient is the subject of the diagnostic administration, the age, body weight, general health, sex, and diet of the patient. Such a diagnostically effective amount can be determined routinely by one of ordinary skill in the art having regard to their own knowledge, the prior art, and this disclosure.

Administration of the appropriate pharmaceutical composition, can be carried out using any of the accepted modes of administration of pharmaceutical compositions. Thus, administration can be, for example, orally, buccally (e.g., sublingually), nasally, parenterally, topically, transdermally, vaginally, or rectally, in the form of solid, semisolid, lyophilized powder, or liquid dosage forms, such as, for example, tablets, suppositories, pills, soft elastic and hard gelatin capsules, powders, solutions, suspensions, or aerosols, or the like, preferably in unit dosage forms suitable for simple administration of precise dosages. The pharmaceutical compositions will generally include a conventional pharmaceutical carrier or excipient and a compound of the invention as the/an active agent, and, in addition, may include other medicinal agents, pharmaceutical agents, carriers, adjuvants, diluents, vehicles, or combinations thereof. Such pharmaceutically acceptable excipients, carriers, or additives as well as methods of making pharmaceutical compositions for various modes or administration are well-known to those of skill in the art. The state of the art is evidenced, e.g., by Remington: The Science and Practice of Pharmacy, 20th Edition, A. Gennaro (ed.), Lippincott Williams & Wilkins, 2000; Handbook of Pharmaceutical Additives, Michael & Irene Ash (eds.), Gower, 1995; Handbook of Pharmaceutical Excipients, A.H. Kibbe (ed.), American Pharmaceutical Ass'n, 2000; H.C. Ansel and N.G. Popovish, Pharmaceutical Dosage Forms and Drug Delivery Systems, 5th ed., Lea and Febiger, 1990; each of which is incorporated herein by reference in their entireties to better describe the state of the art.

As one of skill in the art would expect, the forms of the compounds of the invention utilized in a particular pharmaceutical formulation will be selected (e.g., salts) that possess suitable physical characteristics (e.g., water solubility) that is required for the formulation to be efficacious.

Pharmaceutical compositions suitable for buccal (sub-lingual) administration include lozenges comprising a compound of the present invention in a flavored base, usually sucrose, and acacia or tragacanth, and pastilles comprising the compound in an inert base such as gelatin and glycerin or sucrose and acacia.

Solid dosage forms for oral administration of the compounds include capsules, tablets, pills, powders, and granules. For such oral administration, a pharmaceutically acceptable composition containing a compound(s) of the invention is formed by the incorporation of any of the normally employed excipients, such as, for example, pharmaceutical grades of mannitol, lactose, starch, pregelatinized starch, magnesium stearate, sodium saccharine, talcum, cellulose ether derivatives, glucose, gelatin, sucrose, citrate, propyl gallate, and the like. Such solid pharmaceutical formulations may include formulations, as are well-known in the art, to provide prolonged or sustained delivery of the drug to the gastrointestinal tract by any number of mechanisms, which include, but are not limited to, pH sensitive release from the dosage form based on the changing pH of the small intestine, slow erosion of a tablet or capsule, retention in the stomach based on the physical properties of the formulation, bioadhesion of the dosage form to the mucosal lining of the intestinal tract, or enzymatic release of the active drug from the dosage form.

Liquid dosage forms for oral administration of the compounds include emulsions, microemulsions, solutions, suspensions, syrups, and elixirs, optionally containing pharmaceutical adjuvants in a carrier, such as, for example, water, saline, aqueous dextrose, glycerol, ethanol and the like. These compositions can also contain additional adjuvants such as wetting, emulsifying, suspending, sweetening, flavoring, and perfuming agents.

Rectal administration can be effected utilizing unit dose suppositories in which the compound is admixed with low-melting water-soluble or insoluble solids such as fats, cocoa butter, glycerinated gelatin, hydrogenated vegetable oils, mixtures of polyethylene glycols of various molecular weights, or fatty acid esters of polyethylene glycols, or the like. The active compound is usually a minor component, often from about 0.05 to 10% by weight, with the remainder being the base component.

Generally, a therapeutically effective daily dose is from about 0.001 mg to about 15 mg/kg of body weight per day of a compound of the invention; preferably, from about 0.1 mg to about 10 mg/kg of body weight per day; and most preferably, from about 0.1 mg to about 1.5 mg/kg of body weight per day. For example, for administration to a 70 kg person, the dosage range would be from about 0.07 mg to about 1050 mg per day of a compound of the invention, preferably from about 7.0 mg to about 700 mg per day, and most preferably from about 7.0 mg to about 105 mg per day. Some degree of routine dose optimization may be required to determine an optimal dosing level and pattern.

As used herein, free flowing is defined as that it can be easily processed with conventional pharmaceutical tableting, capsule-filling, or other formulation processes.

As used herein, compressible is defined as that it can be compressed to a tablet, dry granules or other formulations with appropriate mechanical strength and disintegration rates.

The products that use this invention would give better stability as compared to those that use vitamin E TPGS in liquid formulation. This invention provides a broad opportunity for improving dissolution of "water-insoluble" compounds in dosage forms and therefore increase product development opportunity of many previously undevelopable compounds. Compared to blending with water insoluble substrates, this approach provides a better miscibility between vitamin E TPGS and polymers and thereby we can eliminate the processing difficulty encountered with vitamin E TPGS completely. In addition, mixing with water soluble polymers not only accelerates the release of drug compounds but also enhances solublization of compounds. In addition, the following processing properties have been improved by using this invention, they include, flow and compression properties, content uniformity, etc.

The instant surfactant-like material differs from the so called "microemulsions as solid dosage forms" in that the drugs to be loaded do not need to form microemulsions prior to loading into with the active ingredient, thus formulation procedures are much simpler. The instant surfactant-like material is also different from compositions employing Vitamin E TPGS alone as in its original form, VeTPGS has a limited effect on improving overall dissolution, in addition,it is difficult to process. This type of solid dosage forms does not improve the overall dissolution of tables and tablets made from physical mixtures of drug substance with Vitamin E TPGS have higher dissolution than 10 mg SDS tables. Particularly, it is noted that Vitamin E TPGS is very difficult to process due to its waxy nature resulting from low melting point. Our preliminary experiment showed that initial dissolution rate of a model composition can improve tablet bioavailability to about 30% of SEDDs (AUC), which is significantly higher than that of 10 mg SDS tablets (approximately six times higher).

For the mechanistic details of a composition that comprises surfactant-like material which is a homogenous, amorphous or semi-crystalline powder and a poorly soluble therapeutically active agent or a tautomer, , solvate or salt therof and optionally a pharmaceutically acceptable active agent, we hypothesize that dissolution improvement arises from improved mixing of Vitamin E TPGS with drug substance and thereby a better interaction between Vitamin E TPGS and drug substance. This mechanism is feasible for poorly soluble or insoluble drugs.

The surfactant-like material preserves all the manufacturing conveniences and patient compliances of pharmaceutical solid dosage forms with comparable effectiveness to other solubility-improving approaches for insoluble drugs.

The drug loaded powder can be further compressed with disintegrents to tablets or filled in capsules.

Other pharmaceutical ingredients, such as binders, disintegrants, or coating materials, may be preferably used with the present dosage forms. These ingredients include but are not limited to microcrystalline cellulose, croscarmelose sodium, crospovidone, starch, methylcellulose A, sodium alginate, and cellulosephthalate.

The compositions of this invention can be formulated to various solid-dosage forms, such as tablets, coated tablets, hard capsules, and granules using conventional methods.

The invention will be better understood by reference to the Examples which follow.

### EXAMPLES

### Example 1

Preparation of surfactant-like material comprising VeTPGS and VA 64 as the water soluble polymer:
VeTPGS and VA 64 were dissolved in ethanol in 1 to 2 ratios up to 6% followed by heating up the solution to 50 °C on a hotplate with stirring until all the material was completely dissolved. Spray drying process was used to produce VeTPGS/VA 64 solid dispersion using a Buchi Model B-290 spray dryer. 6% ethanol solution of VeTPGS and VA 64 was first prepared before spray drying. During spray drying operation, nitrogen gas was used as inert gas to purge the system thoroughly before pumping liquid. Typically following parameters were used for spray drying using a Buchi Model B-290 spray dryer. Nitrogen pressure of 40 psi was used for atomization and 100% aspiration was used throughout drying period. Pump rate varied from 35 to 50% and inlet temperature was set to 68 °C which resulted in an outlet temperature of 39 or 42 °C depending on pump rate. After spray drying, the material was scraped out of chambers and passed through an 18 mesh screen. For experimental stability batch preparation, Niro Mobile Minor-XP spray dryer was used. The process parameters of Niro process include inlet temperature 69 °C and outlet temperature 39 °C. In addition, both 6% and 20% solid concentration of ethanol solution were used for spray drying.

### Example 2:

Preparation of surfactant-like material comprising VeTPGS and PVP K17 as the water soluble polymer:
VeTPGS/PVP K17 solid dispersion was prepared using the same procedure as in Example 1.

### Example 3:

Incorporating the surfactant-like material into a solid dosage form:
Typically, active agent substance and VeTPGS/VA 64 solid dispersion were triturated in a 1 to 3 ratio (w/w) in a mortar with pestle followed by mixing with mannitol (300 mg mannitol for every 100 mg of active agent substance) in a Turbula mixer at 32 rpm for 12 min. Then, 700mg of drug/dispersion/mannitol mixture and 267 mg of inert blend were weighed out separately for each tablet and mixed on a weigh paper using a spatula followed by transferring the material to a tablet die of 9.3mmX19.1 mm and compressing at a force of 2.7 KN on a Carver press.

**Table 1: The composition of the VeTPGS/VA 64 conventional tablet formulation**

| Component | Weight (mg) |
|---|---|
| Active agent substance | 100 |
| VeTPGS | 100 |
| VA 64 | 200 |
| Mannitol | 300 |
| MCC | 233 |
| Sodium Starch Glycolate | 24 |
| Colloidal Sillicon Dioxide | 8 |
| Mg Stearate | 2 |

Similarly the composition of VeTPGS/PVP K17 conventional tablet formulation was prepared using the same procerdure.

**Table 2: The composition of the VeTPGS/PVP K17 conventional tablet formulations**

| Component | Weight (mg) |
|---|---|
| Active agent substance | 100 |
| VeTPGS | 100 |
| PVP K17 | 200 |
| Mannitol | 300 |
| MCC | 233 |
| Sodium Starch Glycolate | 24 |
| Colloidal Sillicon Dioxide | 8 |
| Mg Stearate | 2 |

Example 4:

### Preparation of tablets containing 50 mg and 150 mg VeTPGS:

To test the effect of the amount of VeTPGS in the formulation on dissolution of formulation, 50 mg and 150 mg VeTPGS tablets were also prepared. For preparing 50 mg VeTPGS tablets, active agent substance and VeTPGS/VA 64 dispersion (100 mg to 150 mg) for each tablet were triturated in a mortar with pestle followed by mixing with 300 mg mannitol and other inert excipients in the same way as preparing 100 mg VeTPGS tablets. Tablets were compressed at a force of 2.7 KN on a Carver press. To incorporate 150 mg VeTPGS into tablet formulation, 100 mg drug substance and 450 mg VeTPGS/VA 64 solid dispersion were mixed and triturated in a mortar with pestle for each tablet followed by mixing with mannitol and inert excipients in the same proportion and same manner as 100 mg VeTPGS tablet. Then, the same procedure used for preparing 100 mg VeTPGS tablet was used to prepare 150 mg VeTPGS tablet. In addition, the modified VeTPGS/PVP K17 conventional tablets were prepared in the same manner as the modified 100 mg VeTPGS/VA 64 conventional tablets.

### Example 5:

### Preparation of VeTPGS/VA64 tablets containing tromethamine (Tris) to further improve in vivo bioavailability:

Tris was incorporated into VeTPGS/VA64 tablet formulation to synergistically enhance dissolution since Tris is a base which can modify the local pH environment of Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.04,6]nonadec-7-ene-4-carboxylic acid which has higher solubility in basic conditions. The composition of the VETPGS/VA64 containing Tris is shown in Table 3. The tablets were prepared similarly as described in Example 3.

**Table 3 Composition of the VeTPGS/VA64/Tris tablet formulation**

| Component | Composition (mg) |
|---|---|
| Active Ingredient | 100 |
| VeTPGS | 100 |
| Va64 | 200 |
| Tris | 150 |
| Mannitol | 300 |
| MCC | 233 |
| Sodium starch glycolate | 24 |
| Colloidal silicon dioxide | 8 |
| MgStearate | 14 |

**Table 4 : Composition of the VeTPGS/VA64/Tris tablet formulation**

| component | Composition mg |
|---|---|
| Active Ingredient | 200 |
| VeTPGS | 100 |
| Va64 | 200 |
| Tris | 150 |
| mannitol | 150 |
| MCC | 90 |
| Sodium starch glycolate | 50 |
| Colloidal silicon dioxide | 8 |
| MgStearate | 2 |

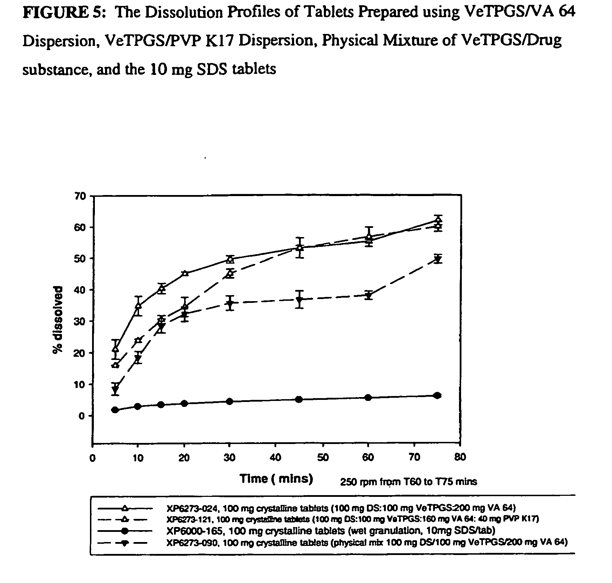

In vitro evaluation of active agent composition with the surfactant-like material vis-à-vis active agent composition with VeTPGS alone:
After incorporation of VeTPGS/VA 64 solid dispersion into tablet formulations, both process and dissolution have been improved possibly due to intimate contacts created between drug molecules and VeTPGS. Fig 5 shows the in vitro dissolution profiles of tablets prepared using VeTPGS/VA 64 solid dispersion and VeTPGS/PVP K17 solid dispersion. In addition, the dissolution profiles of 10 mg SDS tablets and tablets made from the physical mixture of VeTPGS/drug substance are also shown in Figure 5, indicating that tablets prepared using VeTPGS/VA 64 and VeTPGS/PVPk17 solid dispersions have much higher dissolution than those made from the physical mixture and 10 mg SDS tablets. At this point, it can be assumed that dissolution improvement from incorporation of VeTPGS/VA 64 dispersion into tablet formulations is due to an improved mixing of VeTPGS with drug substance and thereby a better interaction between VeTPGS and drug substance.

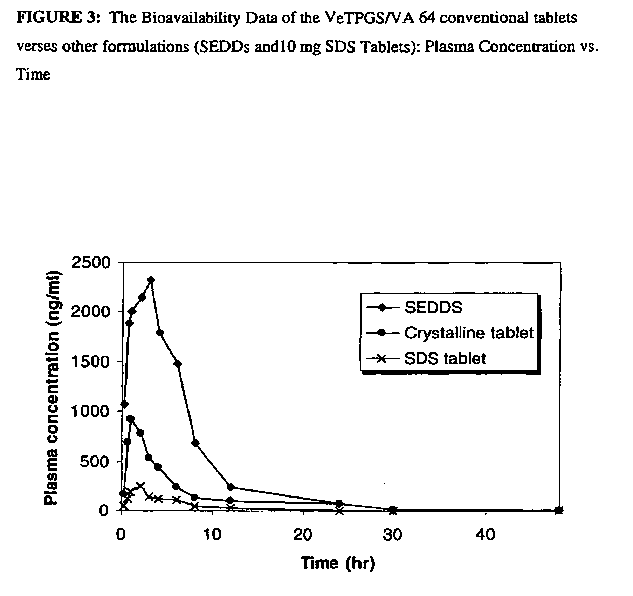

In vivo Bioavailability studies:
The oral bioavailability of tablets prepared using VeTPGS/VA 64 in dogs is displayed n Figure 3. In addition the bioavailability data of 10 mg SDS tablet is shown for comparison. Figure 3 indicates that incorporation of VeTPGS into tablet formulation in form of VeTPGS/VA 64 dispersion can significantly improve the bioavailability of the Compound I(?) over the 10 mg SDS tablets.

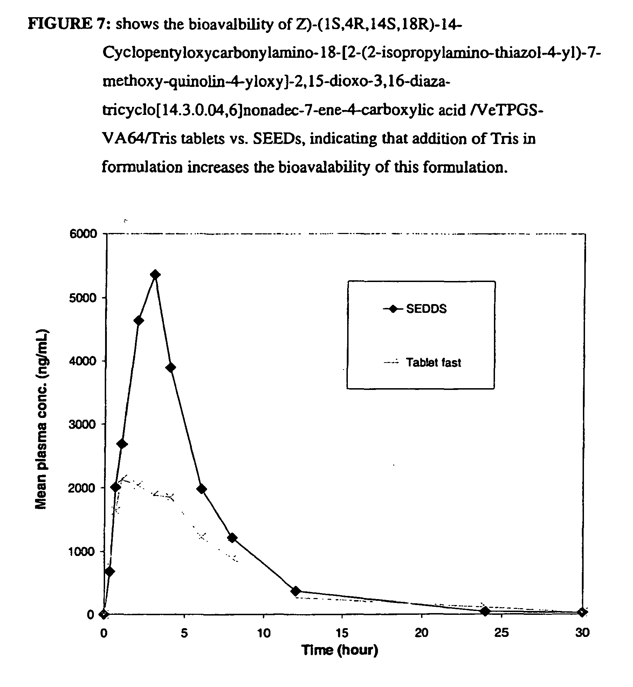

Figure. 7 shows the bioavalbility of Z)-(1S,4R,14S,18R)-14-Cyclopentyloxycarbonylamino-18-[2-(2-isopropylamino-thiazol-4-yl)-7-methoxy-quinolin-4-yloxy]-2,15-dioxo-3,16-diaza-tricyclo[14.3.0.04,6]nonadec-7-ene-4-carboxylic acid /VeTPGS-VA64/Tris tablets vs. SEEDs, indicating that addition of Tris in formulation increases the bioavalability of this formulation.

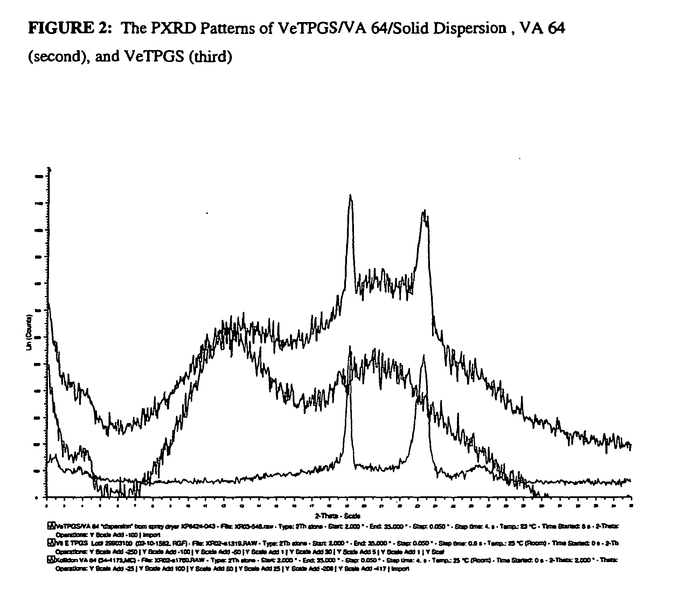

Characterization of physical nature of the surfactant-like material:
To overcome the stickiness of VeTPGS and improve its processing property, VeTPGS was first mixed with polymers of high glass transition temperatures (Tg) at molecular level. Two pharmaceutical polymers: PVP (167 °C) and Kolloidon VA 64 (100 °C) were selected to blend with VeTPGS based on their Tg values and hydrophilic/hydrophobic properties. Figure 2 shows the PXRD patterns of VeTPGS/VA 64 dispersion from spray drying, VeTPGS alone and VA 64 alone, indicating that the solid dispersion of VeTPGS/VA 64 from spray drying is semi crystalline in nature and a portion of VeTPGS crystallized out during the process. This can be confirmed by MDSC thermogram of the solid dispersion where a melting peak for VeTPGS can be observed and indicates that a portion of VeTPGS forms an amorphous phase with VA 64 (Tg ≈ 70 °C). Based on the polarized microscopic examination of some solid dispersion samples, it appears that VeTPGS crystallites distributes uniformly throughout the sample.

## Claims

1. A surfactant-like material comprising Vitamin E TPGS and at least one water soluble polymer and optionally additional carrier or excipients, and which does not contain a therapeutically active agent or a tautomer, solvate or salt thereof

2. The surfactant-like material according to claim 1, which is a homogeneous, amorphous or semi-crystalline powder.

3. The surfactant-like material according to claim 1 or 2, in which the water soluble polymer is chosen from the group of PVP K17, PVP K30 and VA 64.

4. The surfactant-like material according to claim any of the preceding claims, in which the ratio of water soluble polymer to Vitamin E TPGS is the range from about 2:1 to about 15:1.

5. The surfactant-like material according to any of the preceding claims, in which the ratio of water soluble polymer to Vitamin E TPGS is the range from about 2:1 to about 5:1.

6. A method of preparing the surfactant-like material according to any of the preceding claims, comprising the steps of:
(a) dissolving Vitamin E TPGS and a water soluble polymer in a solvent system; and
(b) employing drying technology till the resultant material is a free-flowing powder.

7. A method of preparing the composition comprising surfactant-like material according to claims 2-5 and a poorly soluble active agent or a tautomer, solvate or salt thereof and optionally a pharmaceutically acceptable carriers or excipients comprising the steps of:
a. Dissolving the VeTPGS and the water soluble polymer in a solvent system; and
b. Employing drying technology till the resultant material a free-flowing powder;
c. Mixing the dispersion with a poorly soluble active agentand optionally other excipients followed by a direct compression or by wet granulation and drying; and
d. Optionally followed by mixing granules with other tablet excipients for tableting.

## Patentansprüche

1. Tensidartiges oder -ähnliches Material, umfassend Vitamin-E-TPGS und mindestens ein wasserlösliches Polymer und gegebenenfalls zusätzlich Träger oder Hilfsstoffe, und das kein therapeutisch wirksames Mittel oder ein Tautomer, Solvat oder Salz hiervon enthält.

2. Tensidartiges oder -ähnliches Material nach Anspruch 1, das ein homogenes, amorphes oder semi-kristallines Pulver darstellt.

3. Tensidartiges oder -ähnliches Material nach Anspruch 1 oder 2, wobei das wasserlösliche Polymer ausgewählt ist aus der Gruppe von PVP K17, PVP K30 und VA 64.

4. Tensidartiges oder -ähnliches Material nach irgendeinem der vorhergehenden Ansprüche, wobei das Verhältnis von wasserlöslichem Polymer zum Vitamin-E-TPGS im Bereich von etwa 2:1 bis etwa 15:1 liegt.

5. Tensidartiges oder -ähnliches Material nach irgendeinem der vorhergehenden Ansprüche, wobei das Verhältnis von wasserlöslichem Polymer zum Vitamin-E-TPGS im Bereich von etwa 2:1 bis etwa 5:1 liegt.

6. Verfahren zur Herstellung des tensidartigen oder -ähnlichen Materials nach irgendeinem der vorhergehenden Ansprüche, umfassend die Schritte:
(a) Lösen von Vitamin-E-TPGS und einem wasserlöslichen Polymer in einem Lösungsmittelsystem und
(b) Verwenden von Trocknungstechnologie, bis das resultierende Material ein freifließendes Pulver darstellt.

7. Verfahren zur Herstellung der Zusammensetzung, umfassend ein tensidartiges oder -ähnliches Material nach den Ansprüchen 2 bis 5 und ein schlecht lösliches aktives Mittel oder ein Tautomer, Solvat oder Salz hiervon und gegebenenfalls pharmazeutisch akzeptable Träger oder Hilfsstoffe, umfassend die Schritte:
a. Lösen des VeTPGS und des wasserlöslichen Polymers in einem Lösungsmittelsystem und
b. Verwenden von Trocknungstechnologie, bis das resultierende Material ein freifließendes Pulver darstellt;
c. Mischen der Dispersion mit einem schlecht löslichen aktiven Mittel und gegebenenfalls anderen Hilfsstoffen, gefolgt von einer direkten Kompression oder einer Nassgranulation und Trocknen und
d. gegebenenfalls gefolgt von Mischen der Granulate mit anderen Tablettenhilfsstoffen zur Tablettierung.

## Revendications

1. Matériau de type tensioactif comprenant de la vitamine E TPGS et au moins un polymère hydrosoluble et, éventuellement, un support ou des excipients complémentaires, et qui ne contient pas d'agent thérapeutiquement actif ou tautomère, solvate ou sel de celui-ci.

2. Matériau de type tensioactif selon la revendication 1, qui est une poudre homogène, amorphe ou semi-cristalline.

3. Matériau de type tensioactif selon la revendication 1 ou 2, dans lequel le polymère hydrosoluble est choisi dans le groupe constitué par PVP K17, PVP K30 et VA 64.

4. Matériau de type tensioactif selon l'une quelconque des revendications précédentes, dans lequel le rapport du polymère hydrosoluble sur la vitamine E TPGS est dans la plage d'environ 2:1 à environ 15:1.

5. Matériau de type tensioactif selon l'une quelconque des revendications précédentes, dans lequel le rapport du polymère hydrosoluble sur la vitamine E TPGS est dans la plage d'environ 2:1 à environ 5:1:

6. Méthode de préparation du matériau de type tensioactif selon l'une quelconque des revendications précédentes, comprenant les étapes suivantes :
(a) dissolution d'une vitamine E TPGS et d'un polymère hydrosoluble dans un système de solvants ; et
(b) utilisation d'une technologie de séchage jusqu'à ce que le matériau obtenu soit une poudre fluide.

7. Méthode de préparation de la composition comprenant un matériau de type tensioactif selon les revendications 2 à 5 et un agent actif faiblement soluble ou un tautomère, solvate ou sel de celui-ci et éventuellement des supports ou excipients pharmaceutiquement acceptables, comprenant les étapes suivantes :
a. dissolution de la VeTPGS et du polymère hydrosoluble dans un système de solvants ; et
b. utilisation d'une technologie de séchage jusqu'à ce que le matériau obtenu soit une poudre fluide ;
c. mélange de la dispersion avec un agent actif faiblement soluble et éventuellement d'autres excipients, étape suivie par une compression directe ou par une granulation à sec et un séchage ; et
d. éventuellement suivie par le mélange des granulés avec d'autres excipients de comprimé pour la fabrication de comprimés.
